# EUROPEAN PATENT APPLICATION

(11) **EP 3 967 255 A1**
(43) Date of publication of application: **16.03.2022**
(21) Application number: 21196714.6
(22) Date of filing: 14.09.2021
(51) Int. Cl.: A61B 18/14, A61B 34/30, A61B 34/37, A61B 90/00, A61B 18/00

(54) **BEVELED END EFFECTOR ASSEMBLY**

(30) Priority: 14.09.2020 US 202017019984
(71) Applicant: Covidien LP, Mansfield, MA 02048 (US)
(72) Inventor: ALLEN, James, D, Boulder, CO Colorado 80301 (US)
(74) Representative: Maschio, Antonio

(57) **Abstract**

An end effector assembly for a surgical instrument includes a pair of first and second jaw members movable between a spaced apart configuration and an approximated configuration for grasping tissue therebetween. The first jaw member includes a housing having a substantially flat inwardly facing surface and the second jaw member includes a housing having an electrosurgical cutter disposed along a center thereof and a pair of inwardly facing beveled surfaces extending away from the cutter. An electrically conductive sealing plate is disposed on the housing of the first jaw member. A pair of electrically conductive sealing plates is disposed on the housing of the second jaw member on either side of the electrosurgical cutter, wherein an angle between the substantially flat inwardly facing surface of the first jaw member and the bevel of one of the pair of inwardly facing beveled surfaces of the second jaw member is in the range of about 1 degree to about 20 degrees to encourage tissue sloughing away from the electrosurgical cutter after separation.

## Description

### FIELD

The present disclosure relates to surgical instruments and, more particularly, to electrosurgical instruments for sealing and cutting tissue, and methods of manufacturing same.

### BACKGROUND

A surgical forceps is a pliers-like instrument that relies on mechanical action between its jaw members to grasp, clamp, and constrict tissue. Electrosurgical forceps utilize both mechanical clamping action and energy to heat tissue to treat, e.g., coagulate, cauterize, or seal, tissue. Typically, once tissue is treated, the surgeon has to accurately sever the treated tissue. Accordingly, many electrosurgical forceps are designed to incorporate a knife that is advanced between the jaw members to cut the treated tissue. As an alternative to a mechanical knife, an energy-based tissue cutting element may be provided to cut the treated tissue using energy, e.g., thermal, electrosurgical, ultrasonic, light, or other suitable energy.

### SUMMARY

As used herein, the term "distal" refers to the portion that is being described which is further from a user, while the term "proximal" refers to the portion that is being described which is closer to a user. Further, to the extent consistent, any or all of the aspects detailed herein may be used in conjunction with any or all of the other aspects detailed herein.

Provided in accordance with aspects of the present disclosure is a an end effector assembly for a surgical instrument which includes a pair of first and second jaw members. Once or both of the first and second jaw members are movable between a spaced apart configuration and an approximated configuration for grasping tissue between the first and second jaw members. The first jaw member includes a housing having a substantially flat inwardly facing surface and the second jaw member includes a housing having an electrosurgical cutter disposed along a center thereof and a pair of inwardly facing beveled surfaces extending away from the cutter. An electrically conductive sealing plate is disposed on the housing of the first jaw member and a pair of electrically conductive sealing plates is disposed on the housing of the second jaw member on either side of the electrosurgical cutter. An angle between the substantially flat inwardly facing surface of the first jaw member and the bevel of one or both of the pair of inwardly facing beveled surfaces of the second jaw member is in the range of about 1 degree to about 20 degrees.

In aspects according to the present disclosure, the electrosurgical cutter is raised relative to the electrically conductive sealing plates of the second jaw member. In other aspects according to the present disclosure, the electrosurgical cutter protrudes relative to the electrically conductive sealing plates of the second jaw member in the range of about 0.002 inches to about 0.030 inches. In yet other aspects according to the present disclosure, the electrosurgical cutter maintains a gap proximate the electrosurgical cutter between electrically conductive tissue sealing plates in the range of about 0.001 inches to about 0.006 inches. In aspects according other present disclosure, the electrosurgical cutter may be recessed or flush relative to the electrically conductive sealing plates.

In aspects according to the present disclosure, the outer peripheral surfaces of each of the pair of inwardly facing beveled surfaces of the electrically conductive sealing plates of the second jaw member includes a radius in the range of about 0.007 inches to about 0.020 inches to both facilitate mechanical engagement with the housing of the second jaw member and reduce current concentrations along an edge of the electrically conductive sealing plates of the second jaw member. In other aspects according to the present disclosure, the outer peripheral surfaces of the inwardly facing surface of the electrically conductive sealing plate of the first jaw member includes a radius in the range of about 0.007 inches to about 0.020 inches to both facilitate mechanical engagement with the housing of the first jaw member and reduce current concentrations along an edge of the electrically conductive sealing plate of the first jaw member. In aspects according to the present disclosure, the radius may be non-conductive to reduce current concentrations therealong.

In aspects according to the present disclosure, a gap between opposing electrically conductive sealing plates of the first and second jaw members proximate the electrosurgical cutter is in the range of about 0.001 inches to about 0.006 inches and a gap between the opposing electrically conductive sealing plates proximate opposing edges of the electrically conductive sealing plates of the first and second jaw member is in the range of about 0.003 inches to about 0.020 inches.

In aspects according to the present disclosure, the electrosurgical cutter extends around a distal-most end of the second jaw member to facilitate electrosurgical dissection of tissue. In other aspects according to the present disclosure, the first and second jaw members are configured to close in a tip-biased fashion.

Provided in accordance with aspects of the present disclosure is a an end effector assembly for a surgical instrument which includes a pair of first and second jaw members. One or both of the first and second jaw members is movable between a spaced apart configuration and an approximated configuration for grasping tissue between the first and second jaw members. The first jaw member includes a housing having a substantially flat inwardly facing surface and the second jaw member includes a housing having an electrosurgical cutter disposed along a center thereof and a pair of inwardly facing beveled surfaces extending away from the cutter. The cutter extends around a distal-most portion of the second jaw member and is adapted to connect to an electrosurgical energy source. An electrically conductive sealing plate is disposed on the housing of the first jaw member. A pair of electrically conductive sealing plates is disposed on the housing of the second jaw member on either side of the electrosurgical cutter. An angle between the electrically conductive sealing plate of the first jaw member and one or both of the pair of electrically conductive sealing plates of the second jaw member is in the range of about 1 degree to about 20 degrees.

In aspects according to the present disclosure, the first jaw member may include beveled surfaces while the second jaw member includes flat surfaces with an electrosurgical cutter disposed along a center thereof. In yet other aspects according to the present disclosure, both jaw members include beveled surfaces. In still other embodiments, the first jaw member includes a slot defined therein configured to receive an electrosurgical cutter extending from the second jaw, the cutter being configured to define the gap between jaw members. In yet other embodiments, the cutter may be flush or recessed within one or both jaw members.

In aspects according to the present disclosure, the electrosurgical cutter is raised relative to the electrically conductive sealing plates of the second jaw member. In other aspects according to the present disclosure, the electrosurgical cutter protrudes relative to the electrically conductive sealing plates of the second jaw member in the range of about 0.002 inches to about 0.010 inches. In yet other aspects according to the present disclosure, the electrosurgical cutter maintains a gap proximate the electrosurgical cutter between electrically conductive tissue sealing plates in the range of about 0.001 inches to about 0.006 inches.

In aspects according to the present disclosure, the outer peripheral surfaces of each of the pair of inwardly facing beveled surfaces of the electrically conductive sealing plates of the second jaw member includes a radius in the range of about 0.007 inches to about 0.020 inches to both facilitate mechanical engagement with the housing of the second jaw member and reduce current concentrations along an edge of the electrically conductive sealing plates of the second jaw member. In other aspects according to the present disclosure, the outer peripheral surfaces of the inwardly facing surface of the electrically conductive sealing plate of the first jaw member includes a radius in the range of about 0.007 inches to about 0.020 inches to both facilitate mechanical engagement with the housing of the first jaw member and reduce current concentrations along an edge of the electrically conductive sealing plate of the first jaw member.

In aspects according to the present disclosure, a gap between opposing electrically conductive sealing plates of the first and second jaw members proximate the electrosurgical cutter is in the range of about 0.001 inches to about 0.006 inches and a gap between the opposing electrically conductive sealing plates proximate opposing edges of the electrically conductive sealing plates of the first and second jaw member is in the range of about 0.003 inched to about 0.020 inches.

In aspects according to the present disclosure, the first and second jaw members are configured to close in a tip-biased fashion.

### BRIEF DESCRIPTION OF DRAWINGS

The above and other aspects and features of the present disclosure will become more apparent in view of the following detailed description when taken in conjunction with the accompanying drawings wherein like reference numerals identify similar or identical elements.
FIG. 1 is a perspective view of a shaft-based electrosurgical forceps provided in accordance with the present disclosure shown connected to an electrosurgical generator;
FIG. 2 is a perspective view of a hemostat-style electrosurgical forceps provided in accordance with the present disclosure;
FIG. 3 is a schematic illustration of a robotic surgical instrument provided in accordance with the present disclosure;
FIG. 4A is a side view of the first and second jaw members of the end effector assembly shown in an approximated position;
FIG. 4B is an enlarged view of the area of detail of FIG. 4A.
FIG. 4C is an end view of the first and second jaw members of the end effector assembly of FIG. 4A along section line A-A illustrating an electrosurgical cutting mechanism and a beveled design of the second jaw member to promote tissue separation;
FIG. 5A is a cross-section of a prior art jaw member showing seal plates coated on either side of an electrosurgical cutter; and
FIG. 5B is a cross section of a jaw member according to an embodiment of the present disclosure showing a pair of seal plates adhered to either side of the electrosurgical cutter.

### DETAILED DESCRIPTION

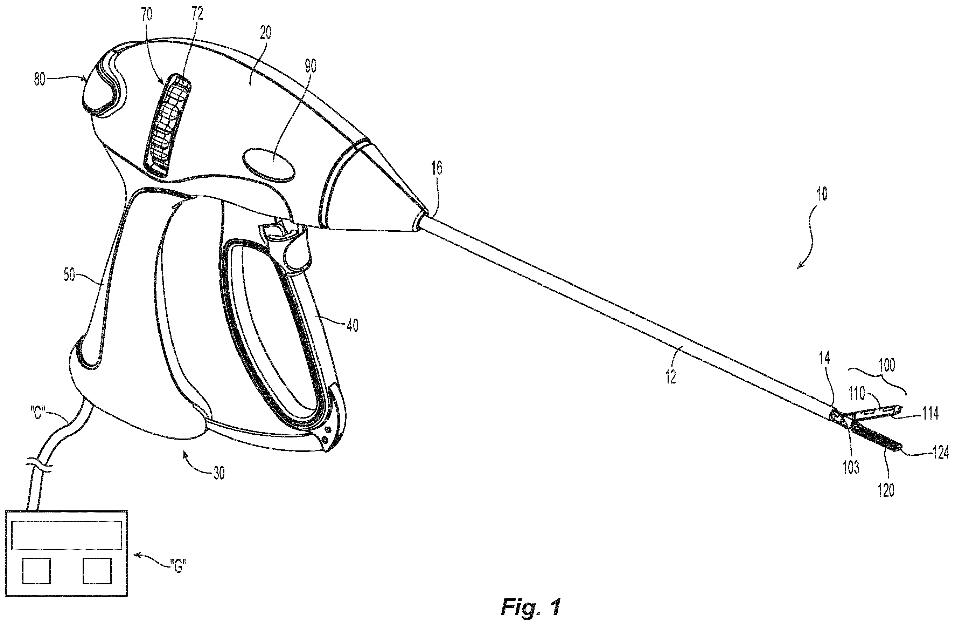

Referring to FIG. 1, a shaft-based electrosurgical forceps provided in accordance with the present disclosure is shown generally identified by reference numeral 10. Aspects and features of forceps 10 not germane to the understanding of the present disclosure are omitted to avoid obscuring the aspects and features of the present disclosure in unnecessary detail.

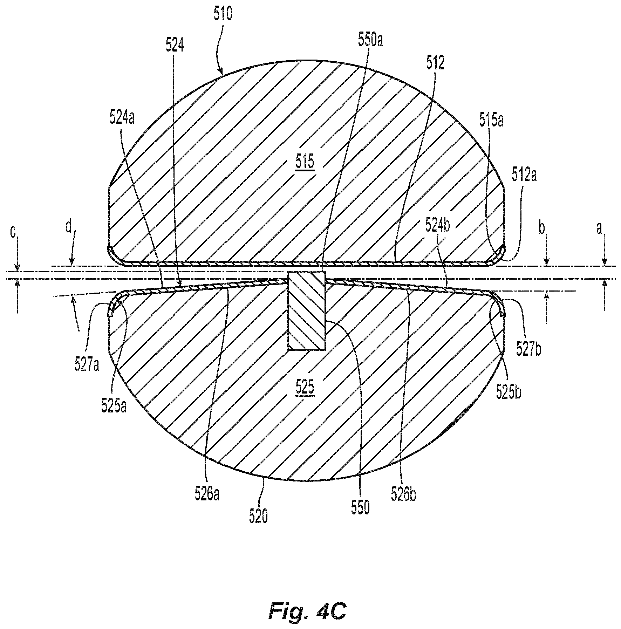

Forceps 10 includes a housing 20, a handle assembly 30, a trigger assembly 60, a rotating assembly 70, a first activation switch 80, a second activation switch 90, and an end effector assembly 100. Forceps 10 further includes a shaft 12 having a distal end portion 14 configured to (directly or indirectly) engage end effector assembly 100 and a proximal end portion 16 that (directly or indirectly) engages housing 20. Forceps 10 also includes cable "C" that connects forceps 10 to an energy source, e.g., an electrosurgical generator "G." Cable "C" includes a wire (or wires) (not shown) extending therethrough that has sufficient length to extend through shaft 12 in order to connect to one or both tissue-treating surfaces 114, 124 of jaw members 110, 120, respectively, of end effector assembly 100 to provide energy thereto. First activation switch 80 is coupled to tissue-treating surfaces 114, 124 and the electrosurgical generator "G" for enabling the selective activation of the supply of energy to jaw members 110, 120 for treating, e.g., cauterizing, coagulating/ desiccating, and/or sealing, tissue. Second activation switch 90 is coupled to thermal cutting element 130 of jaw member 120 and the electrosurgical generator "G" for enabling the selective activation of the supply of energy to thermal cutting element 550 for thermally cutting tissue (FIG. 4C).

Handle assembly 30 of forceps 10 includes a fixed handle 50 and a movable handle 40. Fixed handle 50 is integrally associated with housing 20 and handle 40 is movable relative to fixed handle 50. Movable handle 40 of handle assembly 30 is operably coupled to a drive assembly (not shown) that, together, mechanically cooperate to impart movement of one or both of jaw members 110, 120 of end effector assembly 100 about a pivot 103 between a spaced-apart position and an approximated position to grasp tissue between tissue-treating surfaces 114, 124 of jaw members 110, 120. As shown in FIG. 1, movable handle 40 is initially spaced-apart from fixed handle 50 and, correspondingly, jaw members 110, 120 of end effector assembly 100 are disposed in the spaced-apart position. Movable handle 40 is depressible from this initial position to a depressed position corresponding to the approximated position of jaw members 110, 120. Rotating assembly 70 includes a rotation wheel 72 that is selectively rotatable in either direction to correspondingly rotate end effector assembly 100 relative to housing 20.

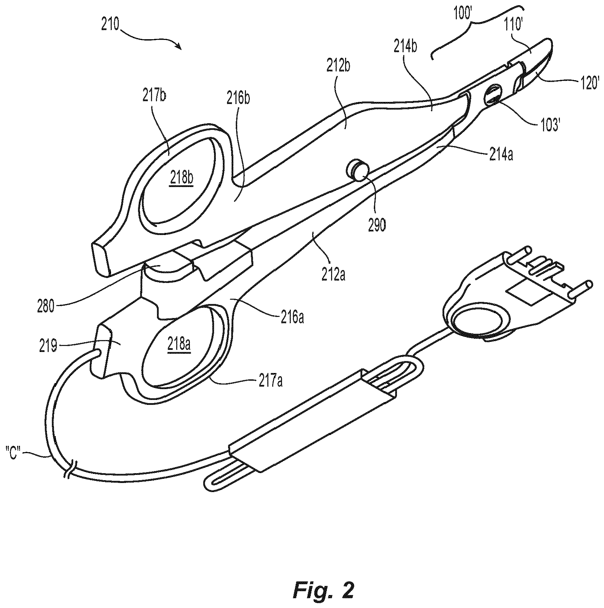

Referring to FIG. 2, a hemostat-style electrosurgical forceps provided in accordance with the present disclosure is shown generally identified by reference numeral 210. Aspects and features of forceps 210 not germane to the understanding of the present disclosure are omitted to avoid obscuring the aspects and features of the present disclosure in unnecessary detail.

Forceps 210 includes two elongated shaft members 212a, 212b, each having a proximal end portion 216a, 216b, and a distal end portion 214a, 214b, respectively. Forceps 210 is configured for use with an end effector assembly 100' similar to end effector assembly 100. More specifically, end effector assembly 100' includes first and second jaw members 110', 120' attached to respective distal end portions 214a, 214b of shaft members 212a, 212b. Jaw members 110', 120' are pivotably connected about a pivot 103'. Each shaft member 212a, 212b includes a handle 217a, 217b disposed at the proximal end portion 216a, 216b thereof. Each handle 217a, 217b defines a finger hole 218a, 218b therethrough for receiving a finger of the user. As can be appreciated, finger holes 218a, 218b facilitate movement of the shaft members 212a, 212b relative to one another to, in turn, pivot jaw members 110', 120' from the spaced-apart position, wherein jaw members 110', 120' are disposed in spaced relation relative to one another, to the approximated position, wherein jaw members 110', 120' cooperate to grasp tissue therebetween.

One of the shaft members 212a, 212b of forceps 210, e.g., shaft member 212b, includes a proximal shaft connector 219 configured to connect forceps 210 to a source of energy, e.g., electrosurgical generator "G" (FIG.1). Proximal shaft connector 219 secures a cable "C" to forceps 210 such that the user may selectively supply energy to jaw members 110', 120' for treating tissue. More specifically, a first activation switch 280 is provided for supplying energy to jaw members 110', 120' to treat tissue upon sufficient approximation of shaft members 212a, 212b, e.g., upon activation of first activation switch 280 via shaft member 212a. A second activation switch 290 disposed on either or both of shaft members 212a, 212b is coupled to the thermal cutting element (not shown, similar to thermal cutting element 550 of jaw member 120 (FIG. 4C)) of one of the jaw members 110', 120' of end effector assembly 100' and to the electrosurgical generator "G" for enabling the selective activation of the supply of energy to the thermal cutting element for thermally cutting tissue.

Jaw members 110', 120' define a curved configuration wherein each jaw member is similarly curved laterally relative to a longitudinal axis of end effector assembly 100'. However, other suitable curved configurations including curvature towards one of the jaw members 110, 120' (and thus away from the other), multiple curves with the same plane, and/or multiple curves within different planes are also contemplated. Jaw members 110, 120 of end effector assembly 100 (FIG. 1) may likewise be curved according to any of the configurations noted above or in any other suitable manner.

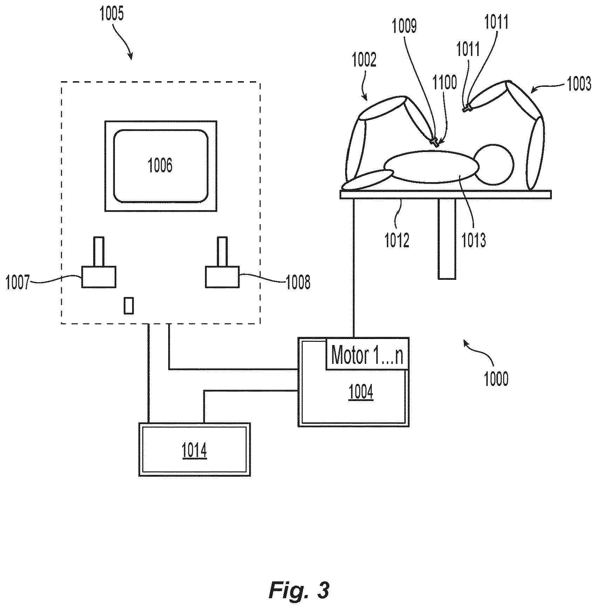

Referring to FIG. 3, a robotic surgical instrument provided in accordance with the present disclosure is shown generally identified by reference numeral 1000. Aspects and features of robotic surgical instrument 1000 not germane to the understanding of the present disclosure are omitted to avoid obscuring the aspects and features of the present disclosure in unnecessary detail.

Robotic surgical instrument 1000 includes a plurality of robot arms 1002, 1003; a control device 1004; and an operating console 1005 coupled with control device 1004. Operating console 1005 may include a display device 1006, which may be set up in particular to display three-dimensional images; and manual input devices 1007, 1008, by means of which a surgeon may be able to telemanipulate robot arms 1002, 1003 in a first operating mode. Robotic surgical instrument 1000 may be configured for use on a patient 1013 lying on a patient table 1012 to be treated in a minimally invasive manner. Robotic surgical instrument 1000 may further include a database 1014, in particular coupled to control device 1004, in which are stored, for example, pre-operative data from patient 1013 and/or anatomical atlases.

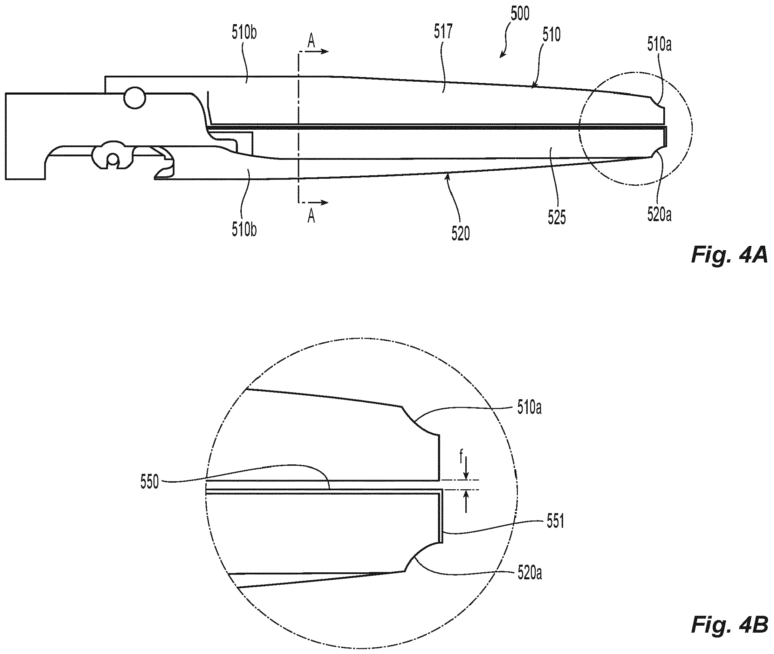

Each of the robot arms 1002, 1003 may include a plurality of members, which are connected through joints, and an attaching device 1009, 1011, to which may be attached, for example, an end effector assembly 1100, 1200, respectively. End effector assembly 1100 is similar to end effector assembly 100 (FIG. 4A), although other suitable end effector assemblies for coupling to attaching device 1009 are also contemplated. End effector assembly 1200 may be any end effector assembly, e.g., an endoscopic camera, other surgical tool, etc. Robot arms 1002, 1003 and end effector assemblies 1100, 1200 may be driven by electric drives, e.g., motors, that are connected to control device 1004. Control device 1004 (e.g., a computer) may be configured to activate the motors, in particular by means of a computer program, in such a way that robot arms 1002, 1003, their attaching devices 1009, 1011, and end effector assemblies 1100, 1200 execute a desired movement and/or function according to a corresponding input from manual input devices 1007, 1008, respectively. Control device 1004 may also be configured in such a way that it regulates the movement of robot arms 1002, 1003 and/or of the motors.

Turning to FIGS. 4A-4C, end effector assembly 500, as noted above, includes first and second jaw members 510, 520. Each jaw member 510, 520 may include a structural housing 515, 525 supporting an electrically conductive tissue-treating plate 512, 524 defining the respective tissue-treating surfaces thereof. In embodiments, tissue-treating plates 512, 524 may be deposited onto jaw housings 515, 525 or jaw inserts (not shown) via any known mechanical process or any known mechanical manufacturing step, vapor deposition, sputtering, overmolding, adhesive, mechanical interfacing components, etc.

Referring in particular to FIGS. 4A-4B, the jaw members 510, 520 are shown in an approximated position wherein the jaw members 510, 520 are generally parallel to one another when approximated. The jaw members 510, 520 are configured in a tip-biased manner such that the tip 510a, 520a of each jaw member 510, 520 touches first when the jaw members 510, 520 are approximated. This also allows the jaw members 510, 520 to pinch tissue as necessary for tissue orientation or dissection. Prior to full actuation of the handle 40, the jaw members close to a generally parallel orientation wherein the jaw members are spaced a distance "f" relative to one another. Upon full actuation, the tips 510a, 520a of each respective jaw members 510, 520 touch in a tip-biased manner. The tip bias "f" may range from about 0.002 inches to about 0.020 inches.

The first jaw member 510 may include beveled surfaces while the second jaw member 520 may include flat surfaces with an electrosurgical cutter 550 disposed along a center thereof. Alternatively, both jaw members 510, 520 include beveled surfaces. The first jaw member 510 may include a slot (not shown) defined therein configured to receive an electrosurgical cutter 550 extending from the second jaw 520, the cutter 550 being configured to define the gap between jaw members 510, 520. The cutter 550 may be flush or recessed within one or both jaw members 510, 520.

FIG. 4C shows a cross section along section line A-A of FIG. 4A detailing the shapes of the jaw members 510, 520 and an electrosurgical cutter 550 along the cross section. More particularly, jaw housing 515 is substantially flat along its cross section and is configured to support sealing plate 512 thereon by any known methods of mechanical attachment. An outer peripheral edge 512a of sealing plate 512 may be shaped to fit a corresponding outer peripheral edge 515a of the jaw housing 515 to facilitate or enhance mechanical engagement or to direct or reduce current concentrations along the surface thereof. The edge 515a radius "e" may be in the range of about 0.007 inches to about 0.020 inches. Sealing plate 512 is disposed opposite cutter 550 and may be energized to an opposite electrical potential to provide a current path through the tissue (when grasped).

Jaw housing 525 is disposed opposite jaw housing 515 along at least partially the length thereof and is configured to house cutter 550 therein. Cutting 550 extends at least partially along the length of jaw housing 525 and includes a tip 551 that extends passed jaw housing tip 520a of jaw housing 520 such that cutter tip 551 is exposed at the distal end of the housing 520 (FIG. 4B). This allows the cutter tip 550 to be used for dissection purposes. Cutter 550 protrudes from or relative to tissue sealing plates 524a, 524b a distance "c" in the range of about 0.002 inches to about 0.010 inches.

Jaw housing 525 includes beveled or tapered surfaces 526a, 526b that each extend away from cutter 550 towards the outer peripheral edges of jaw housing 525. Beveled surfaces 526a, 526b are configure to facilitate cutting and subsequent separation of tissue. More particularly, each beveled surface 526a, 526b includes an angle "d" that facilitates tissue slipping away from or sloughing off of respective tissue sealing plates 524a, 524b on either side of the cutter 550.

The tissue sealing plates 524a, 524b are mechanically engaged to the jaw housing 525 on either side of the cutter 550. More particularly, seal plates 524a, 524b of jaw member 520 are mechanically engaged to jaw housing 525 to at least partially match the angle of the beveled surfaces 526a, 526b. Various angles are envisioned for the beveled surfaces 526a, 526b (and/or seal plates 524a, 524b) and may range from about 1 degree to about 20 degrees depending upon a particular purpose. When the jaw members 510, 520 are approximated, the gap "a" proximate the cutter 550 is in the range of about 0.001 inches to about 0.006 inches and operates as a conventional stop member for vessel sealing purposes.

The outer peripheral edges 527a, 527b of respective tissue sealing plates 524a, 524b may be shaped to fit the corresponding outer peripheral edge 525a, 525b of the jaw housing 525 to facilitate or enhance mechanical engagement or to direct or reduce current concentrations along the surface thereof. The edge 525a, 525b radii "e" may be in the range of about 0.007 inches to about 0.020 inches. The gap "b" proximate the outer peripheral edges, e.g., edge 515a of seal plate 512 and edge 527b of seal plate 524 ranges between about 0.003 inches to about 0.020 inches. The radius may also be made from or coated with a non-conductive material to reduce current concentrations.

Once tissue is sealed by the sealing plates 512, 524 of respective jaw members 510, 520 via actuation of switch 80, the operator may activate switch 90 to energize the cutter 550 (FIGS. 1 and 4C) to electrosurgically cut the tissue. After the tissue is cut, the beveled or tapered configuration of the tissue sealing plates 524a, 524b on either side of the cutter 550 facilitate the tissue falling away from the cutter 550 easing separation thereof.

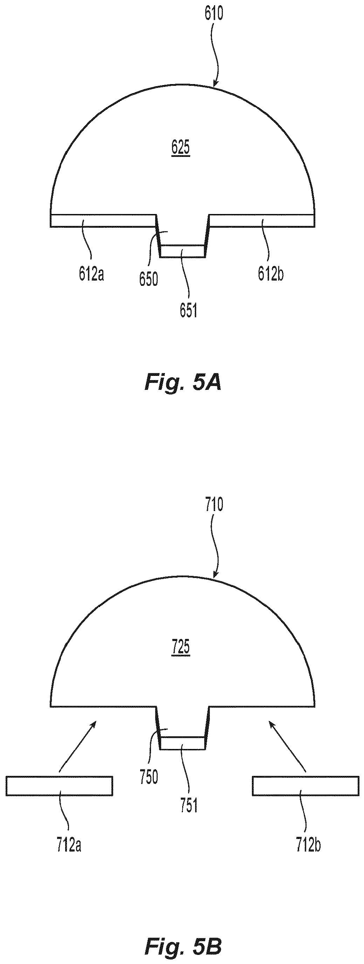

Referring now to FIGS. 5A-5B, an upper jaw member 610 is shown with an electrosurgical cutter 650 disposed along a center thereof between laterally offset sealing plates 612a, 612b. Coating the jaw housing 625 and cutter 650 with electrically conductive sealing and cutting surfaces 612a, 612b and 651 is relatively known and may involve various processes such as vapor deposition, sputtering, etc. Various techniques are described in commonly-owned U.S. Patent Application Serial No. 63,056,113 filed July 24, 2020, the entire contents of which being incorporated by reference herein.

FIG. 5B shows one embodiment according to the present disclosure wherein the sealing plates 712a, 712b are mechanically attached to the housing 725 either side of the cutter 750. More particularly, the seal plates 712a, 712b may be adhered to the housing 725 using a glue or other type of mechanical interface. In embodiments, the seal plates 712a, 712b could be attached using an overmolding process. For example, it is envisioned that adhering the seal plates 712a, 712b to the housing 725 after the housing 725 is formed eliminates complicated manufacturing steps and simplifies the assembly process. An electrically conductive surface 751 may also be adhered (or otherwise attached) to the cutter 750 as well after the housing 725 is formed.

One or both of the jaw members 110, 120 may be configured to include a pinch trim (not shown) between the tissue sealing surfaces and the jaw housing. It is contemplated that designing the pinch trim with certain geometrical configurations may provide additional benefits to facilitate the sealing and/or cutting processes. For example, configuring the pinch trim with certain geometrical features may contain, limit or re-direct smoke during activation of either sealing or cutting. Moreover, certain configurations of the pinch trim may limit the thermal spread or dissipate heat from the cutting element during the cutting process. Still further, certain configurations of the pinch trim may aid in the ejection of tissue once cut.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs: -
1. An end effector assembly for a surgical instrument, comprising:
   a pair of first and second jaw members, at least one of the first and second jaw members movable between a spaced apart configuration and an approximated configuration for grasping tissue between the first and second jaw members, the first jaw member including a housing having a substantially flat inwardly facing surface, the second jaw member including a housing having an electrosurgical cutter disposed along a center thereof and a pair of inwardly facing beveled surfaces extending away from the cutter;
   an electrically conductive sealing plate disposed on the housing of the first jaw member; and
   a pair of electrically conductive sealing plates disposed on the housing of the second jaw member on either side of the electrosurgical cutter, wherein an angle between the substantially flat inwardly facing surface of the first jaw member and the bevel of at least one of the pair of inwardly facing beveled surfaces of the second jaw member is in the range of about 1 degree to about 20 degrees.
2. The end effector assembly according to paragraph 1, wherein the electrosurgical cutter is raised relative to the electrically conductive sealing plates of the second jaw member.
3. The end effector assembly according to paragraph 2, wherein the electrosurgical cutter protrudes relative to the electrically conductive sealing plates of the second jaw member in the range of about 0.002 inches to about 0.010 inches.
4. The end effector assembly according to paragraph 2, wherein the electrosurgical cutter maintains a gap proximate the electrosurgical cutter between electrically conductive tissue sealing plates in the range of about 0.001 inches to about 0.006 inches.
5. The end effector assembly according to paragraph 1, wherein the outer peripheral surfaces of each of the pair of inwardly facing beveled surfaces of the electrically conductive sealing plates of the second jaw member includes a radius in the range of about 0.007 inches to about 0.020 inches to both facilitate mechanical engagement with the housing of the second jaw member and reduce current concentrations along an edge of the electrically conductive sealing plates of the second jaw member.
6. The end effector assembly according to paragraph 5, wherein the outer peripheral surfaces of the inwardly facing surface of the electrically conductive sealing plate of the first jaw member includes a radius in the range of about 0.007 inches to about 0.020 inches to both facilitate mechanical engagement with the housing of the first jaw member and reduce current concentrations along an edge of the electrically conductive sealing plate of the first jaw member.
7. The end effector assembly according to paragraph 1, wherein a gap between opposing electrically conductive sealing plates of the first and second jaw members proximate the electrosurgical cutter is in the range of about 0.001 inches to about 0.006 inches and a gap between the opposing electrically conductive sealing plates proximate opposing edges of the electrically conductive sealing plates of the first and second jaw member is in the range of about 0.003 inched to about 0.020 inches..
8. The end effector assembly according to paragraph 1, wherein the electrosurgical cutter extends around a distal-most end of the second jaw member to facilitate electrosurgical dissection of tissue.
9. The end effector assembly according to paragraph 1, wherein the first and second jaw members are configured to close in a tip-biased fashion.
10. An end effector assembly for a surgical instrument, comprising:
   a pair of first and second jaw members, at least one of the first and second jaw members movable between a spaced apart configuration and an approximated configuration for grasping tissue between the first and second jaw members, the first jaw member including a housing having a substantially flat inwardly facing surface, the second jaw member including a housing having an electrosurgical cutter disposed along a center thereof and a pair of inwardly facing beveled surfaces extending away from the cutter, the cutter extending around a distal-most portion of the second jaw member and adapted to connect to an electrosurgical energy source;
   an electrically conductive sealing plate disposed on the housing of the first jaw member; and
   a pair of electrically conductive sealing plates disposed on the housing of the second jaw member on either side of the electrosurgical cutter, wherein an angle between the electrically conductive sealing plate of the first jaw member and at least one of the pair of electrically conductive sealing plates of the second jaw member is in the range of about 1 degree to about 20 degrees.
11. The end effector assembly according to paragraph 10, wherein the electrosurgical cutter is raised relative to the electrically conductive sealing plates of the second jaw member.
12. The end effector assembly according to paragraph 11, wherein the electrosurgical cutter protrudes relative to the electrically conductive sealing plates of the second jaw member in the range of about 0.002 inches to about 0.010 inches.
13. The end effector assembly according to paragraph 11, wherein the electrosurgical cutter maintains a gap proximate the electrosurgical cutter between electrically conductive tissue sealing plates in the range of about 0.001 inches to about 0.006 inches.
14. The end effector assembly according to paragraph 10, wherein the outer peripheral surfaces of each of the pair of inwardly facing beveled surfaces of the electrically conductive sealing plates of the second jaw member includes a radius in the range of about 0.007 inches to about 0.020 inches to both facilitate mechanical engagement with the housing of the second jaw member and reduce current concentrations along an edge of the electrically conductive sealing plates of the second jaw member.
15. The end effector assembly according to paragraph 14, wherein the outer peripheral surfaces of the inwardly facing surface of the electrically conductive sealing plate of the first jaw member includes a radius in the range of about 0.007 inches to about 0.020 inches to both facilitate mechanical engagement with the housing of the first jaw member and reduce current concentrations along an edge of the electrically conductive sealing plate of the first jaw member.
16. The end effector assembly according to paragraph 10, wherein a gap between opposing electrically conductive sealing plates of the first and second jaw members proximate the electrosurgical cutter is in the range of about 0.001 inches to about 0.006 inches and a gap between the opposing electrically conductive sealing plates proximate opposing edges of the electrically conductive sealing plates of the first and second jaw member is in the range of about 0.003 inched to about 0.020 inches.
17. The end effector assembly according to paragraph 10, wherein the first and second jaw members are configured to close in a tip-biased fashion.

## Claims

1. An end effector assembly of a surgical instrument, comprising:
a first jaw member including:
a first structural jaw;
a first internal spacer disposed on the first structural jaw, the first internal spacer including a first wing extending from a side edge thereof in spaced relation relative to a side surface of the first internal spacer;
a first electrically-conductive plate disposed on the first internal spacer, the first electrically-conductive plate having a first tissue contacting surface defining a first longitudinally extending knife channel therethrough; and
a first outer housing disposed about the first internal spacer, a portion of the first structure jaw, and a portion of the first electrically-conductive plate; and
a second jaw member pivotably coupled to the first jaw member.

2. The end effector assembly of claim 1, further including a first electrical lead wire attached to a portion of the first electrically-conductive plate positioned over the first wing of the first internal spacer.

3. The end effector assembly of claim 1 or 2, wherein the second jaw member includes:
a second structural jaw;
a second internal spacer disposed on the second structural jaw, the second internal spacer including a second wing extending from a side edge thereof in spaced relation relative to a side surface of the second internal spacer;
a second electrically-conductive plate disposed on the second internal spacer, the second electrically-conductive plate having a second tissue contacting surface defining a second longitudinally extending knife channel therethrough; and
a second outer housing disposed about the second internal spacer, a portion of the second structure jaw, and a portion of the second electrically-conductive plate.

4. The end effector assembly of claim 3, further including a second electrical lead wire attached to a portion of the second electrically-conductive plate positioned over the second wing of the second internal spacer.

5. The end effector assembly of claim 3, wherein the second internal spacer includes a partially-cylindrical cut-out in communication with the second longitudinally extending knife channel defined through the second electrically-conductive plate.

6. The end effector assembly of claim 5, wherein the partially-cylindrical cut-out has a generally D-shaped configuration and is open at a proximal end of the second internal spacer to permit insertion of a knife blade and a knife rod therethrough.

7. The end effector assembly of claim 3, wherein a distal portion of the first structural jaw, the first internal spacer, the first outer housing, and the first electrically-conductive plate form a distal body portion of the first jaw member, and a proximal portion of the first structural jaw forms a proximal flange portion of the first jaw member.

8. The end effector assembly of claim 7, wherein a distal portion of the second structural jaw, the second internal spacer, the second outer housing, and the second electrically-conductive plate form a distal body portion of the second jaw member, and a proximal portion of the second structural jaw forms a proximal flange portion of the second jaw member.

9. The end effector assembly of claim 8, wherein the proximal flange portions of the first and second jaw members are pivotably coupled to one another about a pivot pin.

10. The end effector assembly of claim 9, wherein each of the proximal flange portions includes at least one pivot aperture disposed axially behind the distal body portion of the second jaw member, the pivot apertures aligned for receiving the pivot pin therethrough.

11. The end effector assembly of claim 10, wherein the proximal flange portion of the first jaw member includes a single flange defining a pivot aperture therethrough, and the proximal flange portion of the second jaw member includes a pair of spaced-apart flanges defining aligned pivot apertures therethrough, the single flange of the first jaw member disposed between the pair of spaced-apart flanges of the second jaw member.

12. The end effector assembly of claim 7, wherein the proximal flange portion of the first jaw member further includes a cam slot defined therethrough, the cam slot configured to slidably receive a cam pin.

13. The end effector assembly of claim 12, wherein the proximal flange portion of the first jaw member includes a lip extending around the cam slot.

14. The end effector assembly of claim 13, wherein the lip extends tangentially outward from a side surface of a plate-shaped flange of the proximal flange portion.

15. A surgical instrument, comprising:
an end effector assembly as claimed in any one of claims 1 to 14 including:
a first jaw member including:
a first structural jaw;
a first internal spacer disposed on the first structural jaw, the first internal spacer including a first wing extending from a side edge thereof in spaced relation relative to a side surface of the first internal spacer;
a first electrically-conductive plate disposed on the first internal spacer, the first electrically-conductive plate having a first tissue contacting surface defining a first longitudinally extending knife channel therethrough; and
a first outer housing disposed about the first internal spacer, a portion of the first structure jaw, and a portion of the first electrically-conductive plate; and a second jaw member pivotably coupled to the first jaw member; and
a shaft extending proximally from the end effector assembly, the shaft including a distal segment within which proximal flange portions of the end effector assembly are disposed, the first and second jaw members pivotably coupled to one another and the distal segment of the shaft via a pivot pin extending through the proximal flange portions and the distal segment.
